# EUROPEAN PATENT APPLICATION

(11) **EP 2 708 601 A1**
(43) Date of publication of application: **19.03.2014**
(21) Application number: 12006488.6
(22) Date of filing: 14.09.2012
(51) Int. Cl.: C12N 15/113, A61K 31/713, A61P 9/00

(54) **Treatment of heart disease through modulation of hypoxia induced eRNA activity**

(71) Applicant: ETH Zürich, 8092 Zürich (CH)
(72) Inventor: Krek, Wilhelm, 8704 Herrliberg (CH); Krishnan, Jaya, 8047 Zürich (CH); Fankhauser, Niklaus, 3011 Bern (CH)

(57) **Abstract**

The invention relates to targeted RNA interference (RNAi) for the prevention and treatment of cardiomyopathy by inhibiting the expression of the enhancer RNA 22 (eRNA-22). An RNAi agent, a nucleic acid vector encoding a RNAi agent, a virus carrying such vector and a cell capable of producing such virus are provided.

## Description

The present invention relates to RNA interference (RNAi) agents targeting the enhancer RNA 22, (eRNA-22), a nucleic acid vector encoding such RNAi agent, a virus carrying a nucleic acid vector encoding such RNAi agent, or a cell capable of producing a virus carrying a nucleic acid vector encoding such RNAi agent for the prevention and treatment of cardiomyopathy.

Pathologic stress, such as oxygen insufficiency (hypoxia) in mammalian cells facilitates the accumulation and activation of the transcription factor hypoxia-inducible factor 1 (Hif1), a key regulator of transcriptional response to hypoxia. Hif1 plays a pivotal role in the pathophysiology of cancer, cardiovascular diseases and chronic lung diseases.

Cardiomyopathy is a disease of the heart muscle with a decreased function of the myocardium. Cardiomyopathy leads to heart failure, dyspnea, peripheral edema, irregular heart beat and sudden cardiac death. Several types of cardiomyopathy with several causes, such as genetic, acquired, metabolic, inflammatory or endocrine causes, are known.

Hif1 is a heterodimer consisting of the subunits Hif1α and Hif1β. The activity of Hif1 depends on the expression and activity of its α-subunit. Furthermore, Hif1α function is a central and required component of pathologic cardiac growth and contractile dysfunction in various mouse models of human cardiomyopathy. Additionally, *in vivo* ectopic Hif1α activation in cardiomyocytes is sufficient to induce spontaneous pathologic, and not physiologic, cardiac hypertrophy, indicating a key function for Hif1α in the induction and maintenance of cardiac pathologic growth and dysfunction in heart disease. The protein level of Hif1α is increased in samples of patients suffering from cardiomyopathy, leading to the conclusion that the accumulation or stabilisation of this factor promotes disease progression.

The modulation of Hif1 activity has not been explored for the treatment of these diseases.

Enhancer sequences ("enhancers" in the following) are known for their role in activating or enhancing the transcription of their respective target genes far away on the chromosome in a time-dependent and tissue-specific way. Enhancers are highly abundant in the mammalian genome.

Enhancer function itself is managed by regulatory processes leading to tissue-specific and context-dependent patterns of gene expression in the affected cells. During these processes transcription factors regulate, in part, the amplitude and the frequency of the expression of the target genes.

The enhancer regions produce non-coding enhancer RNA (eRNA) molecules that influence the expression of the targeted genes. These eRNAs represent the mechanistic link of time-dependent and tissue-specific gene expression by sequences or segments of chromosomal DNA that amplify or enhance target genes.

eRNA-22 was previously identified (De Santa et al., 2010; PLoS Biol 8, e1000384; SEQ ID 02; chr16:18896739-18896739 / gi|224514941:18896739-18896739 Homo sapiens chromosome 16 genomic contig, GRCh37.p5 Primary Assembly).

RNA interference (RNAi) can be found in many eukaryotes and is defined as a process in cells that regulates the activity of targeted genes on the transcriptional level. Small RNA molecules, such as small interfering RNAs (siRNA) or microRNAs (miRNAs), interact sequence specific with complementary mRNAs of targeted genes, leading to the degradation of this mRNA and thereby preventing the production of the corresponding protein.

The art of silencing or "knocking down" genes, by degradation of mRNA or other effects, is well known. Examples of technologies developed for this purpose include siRNA, miRNA, shRNA, shmiRNA, and dsRNA. A comprehensive overview of this field can be found in Perrimon et al., Cold Spring Harbour Perspectives in Biology, 2010, 2, a003640. Furthermore, ss-siRNA may be used (see Corey et al., Cell (2012) vol. 150 (5) pp. 895-908).

siRNAs can be delivered to the target cell exogenously or expressed endogenously in form of short hairpin RNAs (shRNAs). After expression, these shRNAs are processed by the enzyme Dicer into functional siRNAs (as described in Dallas A, Vlassov AV. RNAi: a novel antisense technology and its therapeutic potential. Med Sci Monit. 2006 Apr;12(4):RA67-74. and references cited therein).

The objective of the present invention is to provide means and methods that allow the prevention and treatment of cardiomyopathy. This objective is attained by the subject matter of the independent claims.

The present invention provides means and methods for the prevention or treatment of cardiomyotpathy by using an RNA interference (RNAi) agent directed against Hif1α-induced eR-NA sequences, in particular against the enhancer RNA 22.

The results presented herein demonstrate a role of eRNA in cellular physiology and show the impact of eRNA function on the pathologic growth, metabolic and contractile reprogramming associated with transition to cardiomyopathy.

The work performed in deriving the present invention provided a number of hypoxia-induced Hif1α-dependent eRNAs. These identified Hifla-regulated eRNAs exhibit sequence and positional conservation between human and mouse genomes, for both the eRNA and their flanking genes. Surprisingly, a subset of these eRNAs are up-regulated in response to pathologic cardiac stressors both *in vitro* and *in vivo.* For some of these eRNAs a functional role in the development and maintenance of the phenotype of cardiomyopathy *in vitro* has been ascertained.

Hif1α-regulated eRNAs were identified through a combination of bioinformatic analysis and experimental validation. By integrating genome-wide hypoxia-induced Hif1α-dependent gene expression data in cardiomyocytes (Jaya Krishnan, Nick Fankhauser and Wilhelm Krek, unpublished data), with lists of identified eRNAs from selected publications (Blow et al., 2010; Nat Genet 42, 806-810; De Santa et al., 2010, PLoS Biol 8, e1000384; Orom et al., 2010, Cell 143, 46-58), 17 putative Hif1α-dependent eRNAs. These eRNAs are conserved in terms of sequence and positional homology between the human and mouse genomes, and are induced in vitro and in vivo in response to pathologic cardiac stressors. Next, functional assays encompassing leucine-incorporation (readout for cardiomyocyte growth and hypertrophy), contractility (readout for contractile dysfunction) and SeaHorse metabolic analysis (readout for changes in cardiomyocyte metabolism), were performed to determine the impact of knockdown specific Hif1α-regulated eRNAs on the pathologic hypertrophic phenotype. The schematic below shows an overview of the process taken to identify Hif1α -regulated heart disease associated eRNAs.

To demonstrate the approach of targeting Hif1α-dependent eRNAs for therapy of cardiomypathy, inhibition of eRNA-22 by RNAi was selected as a (non-limiting) example.

The results contained herein demonstrate that hypoxia promotes eRNA-22 expression via Hif1α. eRNA-22 exhibits a Hif1α-dependent pattern of induction, as indicated by its downregulation upon infection with lentiviruses bearing short hairpin RNAs (shRNAs) targeting Hif1α (shHif1α) and causing RNA interference (RNAi) of Hif1α in cardiomyocytes exposed to hypoxia. The expression of the non-coding eRNA-22 is Hif1α-dependent and plays a functional role in the development and maintenance of the phenotype of cardiomyopathy.

In an *in vivo* model of cardiomyopathy, it could be shown that the expression of eRNA-22 is significantly increased. Isoprotenerol stimulation induces pathologic concentric cardiac hypertrophy, metabolic reprogramming and contractile dysfunction, in part through Hif1α activation and function. Ventricular biopsies of mice subjected to isoproterenol stimulation exhibit significantly increased expression of eRNA-22.

Furthermore, it was found that eRNA-22 has a functional role in the development and maintenance of the phenotype of cardiomyopathy in response to pathologic stress. The knockdown of eRNA-22 via RNAi inhibits Hif1α-induced cardiomyocyte growth induced by ectopic Hif1α expression. RNAi can, by way of non-limiting example, be provided by lentiviral shRNAs targeting eRNA-22 (e.g., sh22-3). sh22-3 inhibits the metabolic reprogramming induced by ectopic Hif1α expression, leading to the maintenance (and augmentation) of fatty acid oxidation and downregulation of glycolysis, both indicators for pathologic stress.

Without whishing to be bound by theory, the data provided herein indicate that the inhibition of pathologic stress-induced Hif1α-dependent eRNA activity by RNAi allows context-dependent and tissue-specific regulation of cardiomyopathy associated coding gene expression. Down-regulation of eRNA-22 in cells exposed to physiologic stress, such as hypoxia, inhibits the metabolic reprogramming induced by ectopic Hif1α expression, leading to the maintenance of cell function.

The data herein provide evidence for the impact of Hif1α-regulated eRNAs in the modulation of development and maintenance of cardiomyopathy and show the importance of eRNAs function in mediating the pathologic growth, and metabolic and contractile dysfunction associated with heart disease.

In one aspect of the invention, an RNAi agent directed against the enhancer RNA 22 (eRNA-22, SEQ ID 01 or SEQ ID 02) for use in a method to prevent or treat cardiomyopathy is provided. SEQ ID 01 is based on homology alignment with the 'coding' region of mouse eRNA-22 (SEQ ID 06). Additionally, SEQ ID 01 includes upstream and downstream sequences flanking the homologous regions, to take into account 5' transcriptional regulatory sequences and 3' stability and regulatory sequences, respectively. These flanking regions provide additional sites to target human eRNA-22. The transcribed eRNA 22 is given as SEQ ID 02.

An RNAi agent or "ribonucleotide based therapeutic agent" in the context of the present invention refers to a ribonucleotide oligomer that causes the degradation of its enhancer RNA (eRNA) target sequence. The target sequence is eRNA 22.

In some embodiments, the RNAi agents of the invention comprise, or consist of,
- a single-stranded or double-stranded interfering ribonucleic acid oligomer or precursor thereof, comprising a sequence tract complementary to said enhancer RNA molecule; or
- a single-stranded or double-stranded antisense ribonucleic or deoxyribonucleic acid, comprising a sequence tract complementary to said enhancer RNA molecule.

In some embodiments, the sequence tract complementary to said enhancer RNA molecule is a contiguous sequence tract 7, 8, 9, 10, 11, 12, 13, 14, 15, 16, 17, 18, 19, 20, 21 or 22 nucleotides in length.

In some embodiments, the RNAi agents of the invention include, but are not limited to, small interfering RNAs (siRNAs), short hairpin RNAs (shRNAs), microRNAs and non-coding RNAs or the like, Morpholinos (phosphodiamidate morpholino oligomers) and Dicer substrate siRNAs (DsiRNAs, DsiRNAs are cleaved by the RNAse III class endoribonuclease dicer into 21 - 23 base duplexes having 2-base 3'-overhangs), UsiRNAs (UsiRNAs are duplex siRNAs that are modified with non-nucleotide acyclic monomers, termed unlocked nucleobase analogs (UNA), where the bond between two adjacent carbon atoms of ribose is removed); self-delivering RNAs (sdRNAs) including rxRNA™ (RXi Pharmaceuticals, Westborough, MA, USA).

In some embodiments, the RNAi agents of the invention comprise analogues of nucleic acids such as phosphotioates, 2'O-methylphosphothioates, peptide nucleic acids (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). The hybridizing sequence may be composed partially of any of the above nucleotides, with the rest of the nucleotides being "native" ribonucleotides occurring in nature, or may be mixtures of different analogues, or may be entirely composed of one kind of analogue.

In one embodiment, the RNAi agent of the invention comprises or consists of a polynucleotide, which specifically hybridizes to eRNA-22 (SEQ ID 01 or SEQ ID 02) and down-regulates expression of this eRNA. As eRNA-22 has a length of more than one thousand nucleotides, several or different target sequences for the hybridization of the RNAi agent are feasible. In some embodiments, the RNAi agent of the invention is designed to target regions within the eRNA-22 sequence bearing less than 30% homology to known coding genes.

In one embodiment, the RNAi agent of the invention comprises or consists of a polynucleotide, which comprises a nucleotide sequence complementary to eRNA-22 (SEQ ID 01 or SEQ ID 02). As used herein, the term "complementary nucleotide sequence," also known as an "antisense sequence," refers to a sequence of a nucleic acid that is completely complementary to the sequence of eRNA-22. Herein, nucleic acid molecules are provided that comprise a sequence complementary to at least about 15, 16, 17, 18, 19, 20, 21, 22, 23, 24, or 25 nucleotides.

In one embodiment of the invention, one or more RNAi agents directed against eRNA-22, varying in the targeted sequence or the type of agent, may be combined.

In some embodiments, the amount or concentration of the targeted eRNA-22 transcripts in a target cell is reduced by at least 10 %, preferably by about 50 % and more preferably by 80 % or more. The amount of RNA transcripts in the respective cells or tissues can be detected, by way of non-limiting example, by chromatographical methods, such as high performance liquid chromatography (HPLC), or polymerase chain reaction (PCR), such as specific and quantitative real time PCR using TaqMan probes.

In some embodiments, the RNAi agent is a short hairpin RNA (shRNA). shRNAs consist of a duplex region and a loop region.

In some embodiments the shRNA is a lentivirally expressed RNA.

In some embodiments, the shRNA comprises, or consists of, the sequence of SEQ ID 03 (sh22-1). In some embodiments, the shRNA comprises, or consists of, the sequence of SEQ ID 04 (sh22-2). In some embodiments, the shRNA comprises, or consists of, the sequence of SEQ ID 05 (sh22-3). In some embodiments, the shRNA comprises, or consists of, the human homologue of sequence SEQ ID 07 (murine sh22-1). In some embodiments, the shRNA comprises, or consists of, the human homologue of sequence SEQ ID 08 (murine sh22-2). In some embodiments, the shRNA comprises, or consists of, the human homologue of sequence SEQ ID 09 (murine sh22-3). All shRNA sequences are given as the DNA coding /sense strand sequence.

According to another aspect of the invention, an expression cassette is provided for use in a method for the prevention or treatment of cardiomyopathy. This expression cassette comprises a polynucleotide (RNA or DNA) sequence that encodes an RNAi agent as described above in general terms or as any of the particular embodiments. Alternatively, the expression cassette encodes a precursor of such RNAi agent. The expression cassette further comprises a promoter sequence operable in a mammalian cell for control of the encoded RNAi agent.

In one embodiment, expression of the RNAi agent, or a precursor thereof, is under the control of a RNA polymerase III (Pol III) or RNA polymerase II (Pol II) promoter. Promoters of Pol III are, by way of non-limiting example, the U6 or the H1 Promoter. Promoters of Pol II are, by way of non-limiting example, the ubiquitin C (UbC) promoter, the phosphoglycerate kinase (PGK) promoter, the human elongation factor-1α (EF1-α) promoter or the Cytomegalovirus (CMV) promoter.

In some embodiments, the promoter is an inducible promoter. In one embodiment, the promoter is a tissue-dependent promoter. In one embodiment, the promoter can be induced by certain small molecule pharmaceuticals.

According to yet another aspect of the invention, an expression vector comprising an expression cassette according to the previous aspect of the invention is provided for use in a method for the prevention or treatment of cardiomyopathy. The expression vector of the invention facilitates production of an RNAi agent targeting the Hifla-regulated eRNA within the cell. Methods for making and using such expression vectors are known in the art.

In some embodiments, the expression vector is a virus such as, by way of non-limiting example, a retrovirus, an adenovirus or an adeno-associated virus.

According to one embodiment, the vector is a lentivirus.

Depending on the type of virus, the expression vector or expression cassette remains episomal or integrates into the genome of the host cell.

In some embodiments, the virus is a non-replicating (replication-incompetent) virus, leading to integration of the transgene into the host genome after one round of infection without generating infectious viruses.

In one embodiment, an shRNA molecule targeting 22 is expressed from a lentiviral vector. This lentiviral vector is integrated in a non-replicating transducing lentivirus. After transcription in the infected cell, the short transcript folds on itself to form a hairpin structure, which is then processed by cellular nucleases to form a 22-suppressing siRNA.

As used herein, the term "shRNA" (small hairpin RNA) refers to an RNA duplex wherein a portion of the siRNA is part of a hairpin structure (shRNA). In addition to the duplex portion, the hairpin structure may contain a loop portion positioned between the two sequences that form the duplex. The loop can vary in length. In some embodiments the loop is 5, 6, 7, 8, 9, 10, 11, 12 or 13 nucleotides in length. The hairpin structure can also contain 3' or 5' overhang portions. In some aspects, the overhang is a 3' or a 5' overhang 0, 1, 2, 3, 4 or 5 nucleotides in length. In one aspect of this invention, a nucleotide sequence in the vector serves as a template for the expression of a small hairpin RNA, comprising a sense region, a loop region and an antisense region. Following expression the sense and antisense regions form a duplex. It is this duplex, forming the shRNA, which hybridizes to eRNA-22.

An expression vector according to the invention may be used for gene therapy purposes. This includes somatic gene therapy, where cells are isolated from the patient and genetically modified. Subsequently, the cells are given back to the patient. The genetic modification of the cell can be performed using the vector described herein, or a genomically integrated form or part thereof.

According to another aspect of the invention, a host cell having one or several polynucleotide sequence(s) that encode, and enable the host cell to produce, a virus is provided, wherein said virus comprises an expression cassette according to the invention.

Such host cell is also referred to as a packaging cell. One non-limiting example of a mammalian packaging cell is the human cell line HEK-293 (ATCC CRL-11268).

According to some embodiments, a packaging cell of the invention comprises:
- an shRNA expression cassette,
- one or two packaging expression constructs encoding the viral structural genes; and
- a heterologous viral envelope expression cassette.

The packaging cell is capable of producing a virus according to the invention. The virus is released from the cells and may be used to transduce cells for transgene expression of shRNAs.

According to another aspect of the invention, a method for the treatment of a patient suffering from cardiomyopathy is provided, comprising administering to the patient an RNA interference (RNAi) agent, an expression cassette or an expression vector according to the above aspects of the invention, in an amount effective to treat the patient.

The treatment may be for therapeutic purposes. For administration, a RNAi agent or a vector according to the above aspects of the invention is provided in the form of a pharmaceutical preparation comprising the agent or vector, and optionally a pharmaceutically acceptable carrier. The RNAi agent or vector is used in an amount effective against cardiomyopathy. The dosage of the RNAi agent or vector depends upon the species, the patient age, weight, and individual condition, the individual pharmacokinetic data and mode of administration.

According to one embodiment, a pharmaceutical composition for the prevention or treatment of cardiomyopathy is provided, comprising an RNAi agent or an expression vector according to the above aspect of the invention.

Pharmaceutical compositions for enteral administration, such as nasal, buccal, rectal or oral administration, and for parenteral administration, such as subcutaneous, intrahepatic, intraperitoneal or intramuscular administration, particularly intravenous, intra-arterial or intraventricular administration, may be used. The pharmaceutical compositions comprise from approximately 1 % to approximately 95 % active ingredient, preferably from approximately 20 % to approximately 90 % active ingredient.

Optionally the administration of the pharmaceutical composition may be used for local induction of RNAi.

Transdermal/intraperitoneal applications are considered, for example using a transdermal patch, which allows administration over an extended period of time, e.g. from one to twenty days.

Pharmaceutical compositions may be sterilized or comprise excipients, for example preservatives, stabilizers, wetting agents or emulsifiers, solubilizers, viscosity-increasing agents, salts for regulating osmotic pressure or buffers and are prepared in a manner known *per se,* for example by means of conventional dissolving and lyophilizing processes.

According to one embodiment, the pharmaceutical composition optionally further comprises a pharmaceutically acceptable carrier.

According to one embodiment the pharmaceutically acceptable carrier is, by way of non-limiting example, a lipoplex or a polyplex, a nanoparticle or a dendrimer.

According to another aspect of the invention, a dosage form for the prevention or treatment of cardiomyopathy is provided, comprising an RNAi agent or an expression vector according to the above aspects of the invention.

According to another aspect of the invention, a method for the manufacture of a medicament for the prevention or treatment of cardiomyopathy is provided, comprising the use of an RNAi agent or an expression vector according to the above aspect of the invention.

Medicaments according to the invention are manufactured by methods known in the art, especially by conventional mixing, coating, granulating, dissolving or lyophilizing.

Wherever reference is made herein to an embodiment of the invention, and such embodiment only refers to one feature of the invention, it is intended that such embodiment may be combined with any other embodiment referring to a different feature.

The invention is further illustrated, without limitations, by the following figures and examples, from which further features, advantages or embodiments can be derived. The examples do not limit but illustrate the invention.

### Description of the figures

- Fig. 1:: Hypoxia (3 % O₂) promotes eRNA-22 expression via Hif1α (A). Schematic representation of eRNA-22 localization and the putative HRE (B). Hif1α binds to the HRE in the eRNA-22 promoter upon hypoxia exposure (C).
- Fig. 2:: The cross-species conserved HRE in the eRNA-22 promoter is required for its activation (A). Flanking coding genes Smg1 (B) and Syt17 (C) show a similar hypoxia-induced Hif1α-dependent expression pattern as eRNA-22.
- Fig. 3:: Isoproterenol (ISO) promotes cardiac hypertrophy (A) and Hif1α accumulation (B).
- Fig. 4:: Transcription of eRNA-22 (A), and its flanking coding genes Smg1 (B) and Syt17 (C) are upregulated specifically in mice subjected to chronic isoproterenol stimulation.
- Fig. 5:: Lentiviral shRNA targeting eRNA-22 (sh22-3) efficiently downregulates eRNA-22 expression (A), and of flanking genes Smg1 (B) and Syt17 (C), non-silencing shRNA (nsRNA) serves as a control
- Fig. 6:: sh22-3 inhibits Hif1α-induced cardiomyocyte growth, as determined by leucine-incorporation as readout for protein synthesis (A), sh22-3 inhibits the metabolic reprogramming induced by ectopic Hif1α expression, leading to the maintenance (and augmentation) of fatty acid oxidation (FAO) (B) and downregulation of glycolysis (C).

### Examples

### Materials and Methods used:

Chemicals: All chemical were purchased from Sigma unless indicated otherwise.

### Animal breeding and maintenance

All mice were maintained at the Institute of Cell Biology, ETH-Zurich specific pathogen-free facility. Maintenance and animal experimentation were in accordance with the Swiss Federal Veterinary Office (BVET) guidelines.

### Human and mouse ventricular biopsies

Human heart biopsies were generously provided by Wolfgang Linke (University of Munster, Germany) (van Heerebeek et al., 2006, Circulation 113, 1966-1973). Ventricular lysates and biopsies from ISO-treated mice (Mazzolai et al., 1998, Hypertension 31, 1324-1330; Mazzolai et al., 2000, Hypertension 35, 985-991), angiotensin II overexpressing transgenic mice (Mazzolai et al., 2000, ibid.) and their corresponding controls were provided by Thierry Pedrazzini.

### In vivo isoproterenol delivery

Alzet model 2002 mini-osmotic pumps (Alzet) were filled with isoproterenol, and were set to deliver at 30 mg/kg/day for 14 days in littermate control wildtype and Hif1• +/-. Pumps were then implanted into 9-10 weeks old male and female mice under isoflurane anesthesia. At the end of the stimulation phase, the heart contractility was measured in anesthetized animals (Intraperitoneal: 90 mg/kg ketamine, 5 mg/kg xylazine).

### Echocardiography

Echocardiography was performed using the Vevo 660 Ultrasonograph (VisualSonics) as previously described (Barrick et al., 2007, Am J Physiol Heart Circ Physiol 292, H2119-2130).

### Mouse cardiomyocyte isolation and culture

P2-P4 neonatal mouse ventricles were dissected, digested with collagenase (Worthington Biochemical Corporation) and pancreatin (Gibco Laboratories) and cultured in Maintenance medium which contained 20% medium M199, 75% DBSS-K, 4% horse serum, 4mM glutamine, 1% penicillin/streptomycin, 0.1mM phenylepinephrine; (DBSS-K: 6.8g/l NaCl, 0.14mM NaH2PO4, 0.2mM CaCl2, 0.2mM MgSO4, 1 mM dextrose, 2.7 mM NaHCO3) in fibronectin-coated plastic dishes (10µg/ml). The maintenance medium for neonatal mouse cardiomyocyte maintenance was alike but lacked serum. Cultured neonatal mouse cardiomyocytes were treated with phenylepinephrine at a concentration of 100µM, with isoproterenol at 10µM and norepinephrine at 10µM for 72 h just before fixation or before preparing SDS-samples. Mouse cardiomyocytes were transfected with Trogene (Life Technologies) as recommended by the manufacturer.

### Adenoviruses, lentiviruses and plasmid expression constructs

Lentiviral shRNAs were designed in-house and synthesiszed by Microsynth. The Hif1alpha ODD expression construct was generated as described (Huang et al., 1998; Proc Natl Acad Sci USA, 95, 7987-7992).

### Antibodies

Antibodies used for immunoblotting and in the ChIP assay against HIF1• were from Santa Cruz Biotechnology.

### SDS-PAGE and immunoblotting

Dissected hearts were homogenized by freeze slamming and solubilized in a modified SDS sample buffer sonicated and boiled for 5 minutes (Hirschy et al., 2006, Developmental Biology 289, 430-441). Cultured cardiomyocyte lysates were harvested with the modified SDS sample buffer, sonicated and boiled. Protein extracts were resolved on 10 or 15% polyacrylamide minigels (BioRad) and transferred overnight onto nitrocellulose membrane (Amersham). Immunodetection and visualization of signals by chemiluminescence was carried out as described (Hirschy et al., 2006, ibid.).

### Luciferase Promoter assays

1.5kb of the eRNA-22 promoter was amplified from mouse BAC genomic DNA and cloned into the pGL3 luciferase reporter vector (Stratagene). HRE- mutants of the respective promoters were generated by recombinant PCR (Casonato et al., 2004, J Lab Clin Med 144, 254-259; Elion et al., 2007, Current protocols in molecular biology (Ed.:M. Ausubel; Chapter 3, Unit 3 17). This was achieved by generating sense and antisense primers bearing HRE mutations, which were used to amplify the mutant 5' and 3' regions of the promoter, respectively. The 5' and 3' products generated from the respective PCR reactions were then pooled at a 1:1 ratio and reamplified using primers targeting the 5' and 3' ends of the respective promoters. In doing so, HRE mutants of the respective promoters were generated. Sequence integrity of the respective wildtype and mutant promoters were verified by sequencing and BLAST alignment (http://www.ncbi.nlm.nih.gov/blast). The reporter assay was performed by transient cotransfection of the appropriate luciferase reporter, pSV-•-galactosidase (Promega), in the presence of hypoxia or, the HIF1••Pro expression constructs. The Luciferase and •-galactosidase activity was measured using the Luciferase Assay System kit (Promega) as recommended by the manufacturer and analyzed on the MicroLumatPlus LB 96V (luciferase activity: Berthold Technologies) and Spectra MAX 190 plate reader (•-galactosidase activity: Molecular Devices). Mouse cardiomyocytes were transfected with Trogene (Life Technologies) as recommended by the manufacturer.

### Quantitative RT-PCR and ChIPs

RNA was isolated with Trizol (Invitrogen) and cDNA generated using Superscript II (Invitrogen) from individual hearts as recommended by the manufacturer. qPCR reactions were setup as recommended by the manufacturer (Roche) and analyzed on the Roche Light-Cycler 480. Quantification of mtDNA was performed as described (Shen et al., 2004, Am. J. Physiology 287, E896-905). The CHIP assay was performed using material from neonatal mouse cardiomyocytes and the assay performed using the CHIP-IT kit (Active Motif) as recommended by the manufacturer. In silico promoter analyses and alignments were performed using Matlnspector and DiAlignTF (Genomatix).

The murine eRNA-22 sequence (DNA coding/sense strand) is given as SEQ ID 06. Three different shRNA constructs targeting murine eRNA22 are given SEQ ID 07 (sh22-1), SEQ ID 08 (sh22-2) or SEQ ID 09 (sh22-3) (the respective DNA coding/sense strands are given).

### Oxygen consumption measurements

Cellular oxygen consumption was measured using a Seahorse bioscience XF24 analyzer in 24 well plates at 37°C, with correction for positional temperature variations adjusted from four empty wells evenly distributed within the plate. 3T3-L1 cells were seeded at 20000 cells/well 8-10 days prior to the analysis. Once confluence was reached, differentiation was induced and viral infection performed on day 4 of differentiation. Each experimental condition was performed on 10 biological replicates, and performed as recommended by the manufacturer (Seahorse Bioscience).

### Statistical analysis

All statistical analyses and tests were carried out using Excel (Microsoft). Data are given as mean +/- standard deviation. Bars in graphs represent standard errors and significance was assed by two-tailed T test with a P value below 0.05.

### Selection/identification of relevant eRNA

eRNA function is linked to the induction of cis-mediated 5' and 3' coding gene expression. Hence, identified eRNAs from the listed reports were cross-referenced against genome-wide hypoxia-induced Hif1α-dependent cardiomyocyte gene expression datasets for Hif1α-dependent changes in flanking gene expression. Identified regions were validated for positional conservation of the flanking genes and eRNA in human and mouse genomes. Positionally conserved eRNAs, and their flanking genes were then experimentally validated by qPCR in in vitro and in vivo models of pathologic hypertrophy. Potential Hif1α-regulated eRNAs were assessed for conservation of HRE by in silico promoter analysis. Subsequently shRNAs targeting Hif1α-regulated eRNAs were designed and assessed for their capacity to attenuate the maladaptive changes in cardiomyocyte growth, metabolism and contractility that are characteristic with pathologic hypertrophy.

### Tissue specificity of eRNA expression

Expression of eRNA-22 was assessed in multiple mouse tissues including kidney, lung, brain, spleen, liver, skeletal muscle and white adipose tissue. In addition, expression of eR-NA-22 was analysed in multiple immortalised and cancer cell lines. However, expression of *eRNA**-**22* was absent or negligible level both in non-heart tissue, and in the cell lines analysed.

### Example 1: Hypoxia promotes eRNA-22 expression via Hif1α

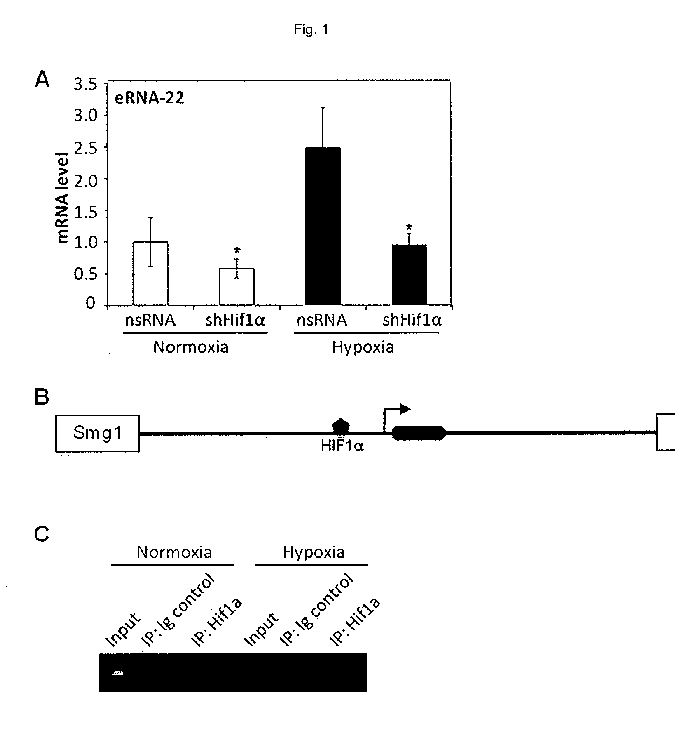

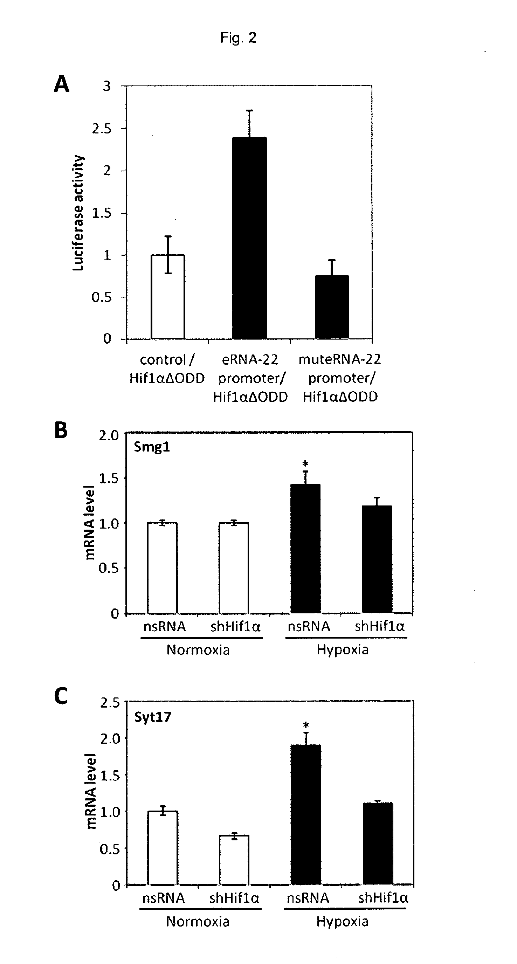

eRNA-22 exhibits a Hif1α-dependent pattern of induction, as indicated by its downregulation upon infection with lentiviruses bearing short hairpin RNAs (shRNAs) targeting Hif1α (shHif1α) in cardiomyocytes exposed to hypoxia (Figure 1A). Scrambled non-silencing RNA (nsRNA) serves as control. eRNA-22 is templated by an intergenic region between the coding genes *C. elegans* smg1-homolog phosphatidylinositol-3-kinase-related kinase (Smg1) and synaptotagmin XVII (Syt17) (Figure 1B). The genomic organisation of the respective flanking genes and eRNA-22 promoter in native chromatin is shown in Figure 1C. Moreover, Hif1α drives eRNA-22 promoter activation via the HRE, as indicated by the failure to activate the eRNA-22 promoter when the HRE motif is altered from the consensus aCGTG HRE motif to aCAAA in cardiomyocytes ectopically expressing Hif1α (Figure 2A). Consistent with the view that eRNAs promote flanking coding gene transcription, Smg1 and Syt17 are concomitantly upregulated upon hypoxia-induced transcriptional induction of eRNA-22, and downregulated upon Hif1α inactivation by shHif1α in cardiomyocytes exposed to hypoxia (Figures 2B and 2C). Despite multiple attempts, the detection of cross-species conserved HREs neither in the Smg1 nor Syt17 promoter by *in silico* analyses of the respective promoters, or experimentally detection of Hif1α binding at the Smg1 or Syt17 locus by ChIP analyzes was not possible(data not shown).

### Example 2: Isoproterenol promotes cardiac hypertrophy and Hif1α accumulation

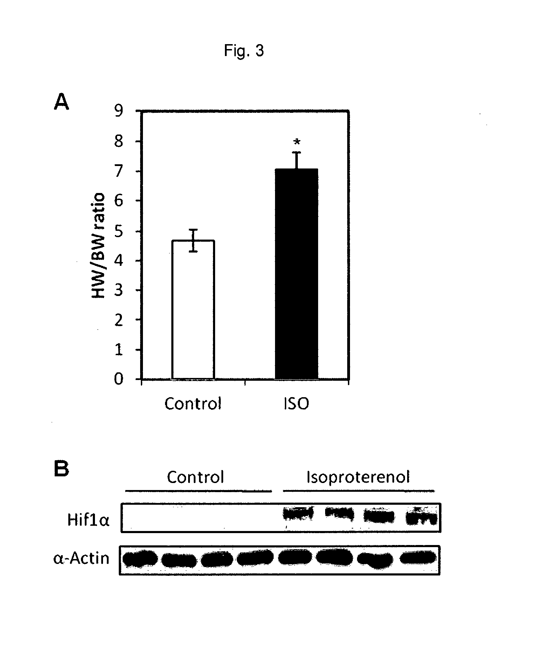

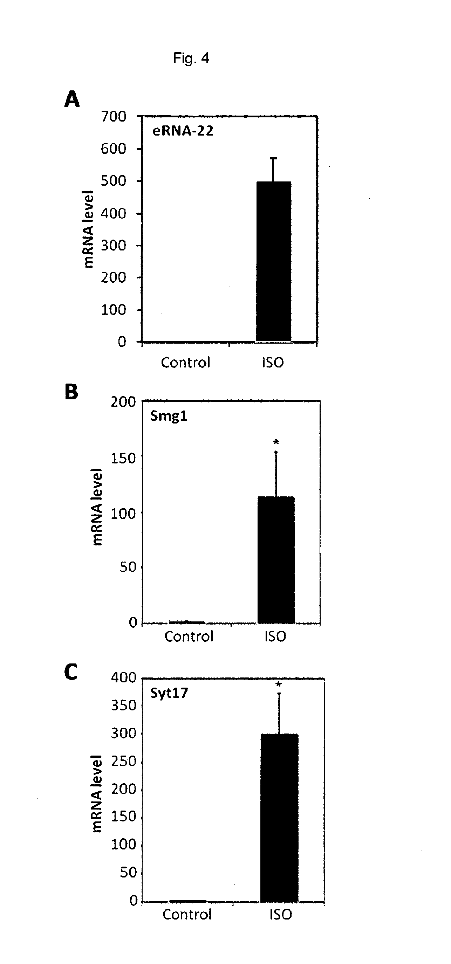

Chronic isoproterenol stimulation induces pathologic concentric cardiac hypertrophy, metabolic reprogramming and contractile dysfunction, in part through Hif1α activation and function (Figure 3A and 3B). Ventricular biopsies of mice subjected to isoproterenol stimulation exhibit significantly increased expression of eRNA-22 (Figure 4A), and of the flanking coding genes Smg1 and Syt17 (Figure 4B and 4C). Given that ventricle-specific Hif1α inactivation inhibits the maladaptive growth and contractile phenotype induced by isoproterenol, these findings suggest a possible contribution of eRNA function to Hif1α-induced pathologic cardiac hypertrophy.

### Example 3: Lentiviral shRNAs targeting eRNA-22 efficiently downregulates eRNA-22 expression

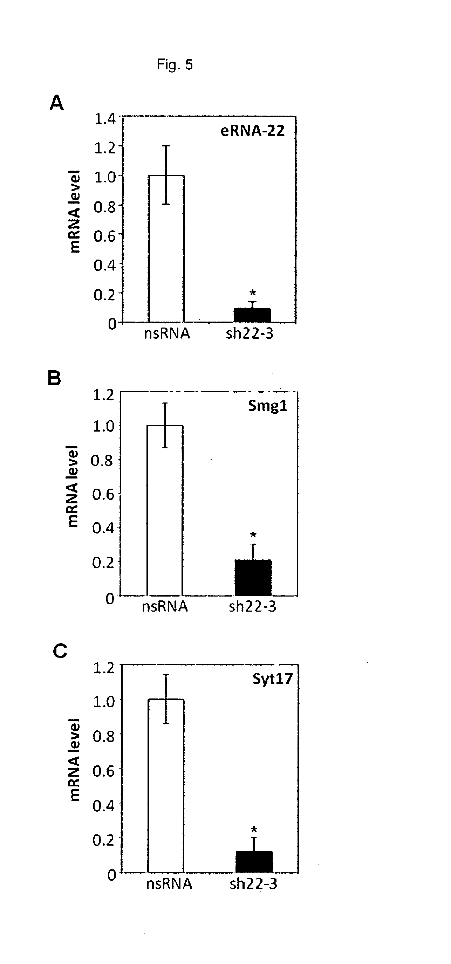

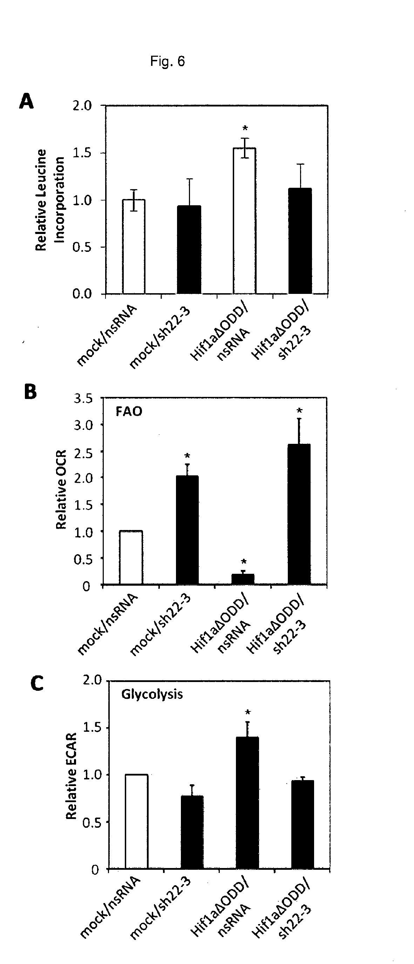

A functional role for eRNA-22 in the development and maintenance of the disease phenotype in response to pathologic stress has been ascertained. In Figure 5A, knockdown of eRNA-22 achieved by infection of short-hairpin RNAs (shRNA) targeting eRNA-22 (sh22-3) led to efficient knockdown of eRNA-22 in cardiomyocytes, concomitant with the inhibition of Smg1 and Syt17 gene expression (Figures 5B and 5C). Importantly, knockdown of eRNA-22 led to the inhibition of cardiomyocyte growth induced by ectopic Hif1α expression (Figure 6A). Simultaneous knockdown of eRNA-22 in cardiomyocytes ectopically expressing Hif1α prevented the inhibition of fatty acid oxidation (FAO) affected by ectopic Hiflα (Figure 6B) and the induction of glycolysis (Figure 6C). These data provide evidence of a functional role for eRNAs in regulating cellular physiology in response to pathologic stress stimuli through cis-mediated control of neighbouring gene expression physiology, and serve as a proof-of-concept validating the importance of eRNAs function in mediating the pathologic growth, and metabolic and contractile dysfunction associated with heart disease.

The pLKO.1 system, in combination with the packaging psPAX2 plasmid and the envelope pMDG.2 plasmid (http://www.addgene.org), was used to generate lentiviruses. Transcription of the shRNA is driven by the human U6 promoter. Information for the respective plasmids can be found at the websites below:
pLKO.1: http://www.addgene.org/10878/
psPAX2: http://www.addgene.org/12260/
pMD2.G: http://www.addgene.org/12259/

## Claims

1. An RNA interference (RNAi) agent directed against the enhancer RNA 22 (eRNA-22, SEQ ID 02) or against SEQ ID 01, for use in a method for the prevention or treatment of cardiomyopathy.

2. The RNAi agent according to claim 1, wherein said RNAi agent is an shRNA or an siRNA.

3. The RNAi agent according to claims 1 or 2, wherein said shRNA is a lentiviral shRNA.

4. The RNAi agent according to claim 2 or 3, wherein said shRNA comprises, or consists of, the sequence of SEQ ID 05 (sh22-3), SEQ ID 03 (sh22-1) or SEQ ID 04 (sh22-2).

5. An expression cassette, comprising a sequence encoding an RNAi agent according to any of claims 1 to 4, or encoding a precursor of said RNAi agent, under the control of a promoter sequence operable in a mammalian cell, for use in a method for the prevention or treatment of cardiomyopathy.

6. An expression cassette according to claim 5, wherein expression of the RNAi agent, or a precursor thereof, is under the control of a RNA polymerase III (Pol III) or RNA polymerase II (Pol II) promoter.

7. An expression vector comprising an expression cassette according to claim 5 or 6, for use in a method for the prevention or treatment of cardiomyopathy.

8. A virus comprising an expression cassette according to claim 5 or 6.

9. A virus according to claim 8 **characterized in that** the virus is a non-replicating virus.

10. A virus according claim 8 or 9 **characterized in that** the virus is a lentivirus.

11. A host cell capable of producing a virus according to one of the claims 8 to 10 for use in a method for the prevention or treatment of cardiomyopathy.

12. A cell according to claim 11 transfected with a mixture of plasmids consisting of:
- an shRNA expression cassette,
- one or two packaging plasmids containing the viral structural genes; and
- a heterologous viral envelope expression cassette.

13. A method for the treatment of a patient suffering from cardiomyopathy comprising administering to said patient an RNAi agent according to one of the claims 1 to 4, an expression cassette according to claim 5 or 6, a vector according to claim 7, or a virus according to one of the claims 8 to 10, in an amount effective to treat said patient.

14. A pharmaceutical composition for the prevention or treatment of cardiomyopathy, comprising an RNAi agent according to one of the claims 1 to 4, an expression cassette according to claim 5 or 6, a vector according to claim 7, or a virus according to one of the claims 8 to 10, for use in a method for the prevention or treatment of cardiomyopathy.

15. A method for the manufacture of a medicament for the prevention or treatment of cardiomyopathy, comprising the use of an RNAi agent according to one of the claims 1 to 4, an expression cassette according to claim 5 or 6, a vector according to claim 7, or a virus according to one of the claims 8 to 10.


SEQUENCE LISTING

<110>  Eidgenoessische Technische Hochschule Zuerich

<120>  Treatment of heart disease through modulation of hypoxia induced
       eRNA activity

<130>  T-12-063

<160>  9

<170>  PatentIn version 3.5

<210>  1
<211>  2543
<212>  DNA
<213>  homo sapiens

<400>  1
gaagctaggt gtttgggagg tgatatggtt tggctgtgtc cccacccaaa tctcatcttg      60

aattgtagct cccatagttc ccacgtgttg taggagagac ccctggtggg agataattga     120

atcatggggg tggtttcccc catactgctc ttgcgatagt gagtaagtct cgagatttga     180

cgattttata acgggttgcc ccttttgctt ggctctcatt ctctcttgtc tgccgccata     240

taagacaagc ttttggcctt ccgccatggt tgtgagacct ccccagccag gtggaactgt     300

gagtccatta aacctctttt tcttcataaa ttacccagtc taaggtatat ctttatcagc     360

agtgtgaaaa tggactaata caggaggtga ttaggtcacg aaggcagagc cctcatgaac     420

aagactagtg tccttataaa agaggcacca agaagctgcc ttgtgccttc cactatgtaa     480

ggacacaaca agaaggtgtc gtgttatctg tgaactagga agtgagccct ccccagatac 540

cgaatctact ggtgccttga ttttggactt attctctaga actatgagaa ataaattcct 600

gttgttatat gccgccagtt tatccgtttt tttttgtttg tttttttttt ttgagatgga 660

gtctcactct gtcgcccagg ctggagtaca atggcacaat ctcagcttac tgcaatcttt 720

gcctcccatg ttcaagagat tctcctgcct cagcctcctg agtagctggg actacaggtg 780

tgcaccacca cacctggcta atttttgtat ttttagtaga gatagggttt cactatgttg 840

gccaggctgg tctcaaactc ctgacctcag gtgatctgcc cgcctcccaa agtgttggga 900

ttacgggtgt gagccaccgc acttggccac tattttttta cagcagcatc catggactga 960

aacaagcatg taccccatgc caggtagtat acatctcagc acttcacagg cagagctcta 1020

atcttcacac atgcttgatg gggaggtatt attattccca ttttacagat taaaatgcca 1080

agcttcagaa tgattgtgtg tattgctaaa gacctggacc cgccttatct tagacttttc 1140

agcaaatgta gttgttcact aagccttatt atttattgtg gcatgtgact tagctgtctc 1200

atttgctaac tcaaaggtac aaaccccttc tcattttttt ttctgtcaac aacagtttcc 1260

agcttataat gggtactcaa atatttagtg aaggaataaa tgagagacac agatatgact 1320

acacacacac acacacaaaa ataacaggca acaaagtgag actccatcct tccaaaaaaa 1380

tgtaaaaata gccaggcata gtggcgtatg cctgtagtct cagctactca ggaggctgag 1440

gtgggaggac cacttgagcc ccagagactg aagctatagt gagctgtgat tgcaccactg 1500

cgctccagcc tgggtgacag agaaaagaga gagagagaga gaaaagccaa aagcccccaa 1560

gtgcattgga ggggaagaat tggatcctaa aaaagtcctg ggatatcatt taatctagaa 1620

gtcattacct cgctggctct agggaaaacg atggcctcag gagtattttg tgtaatttcc 1680

aaattctgca aaataaagtt gtgttagttc ctggcattta taaattgaga aattgcaaat 1740

ggaaatccag attttatttt atttatttat ttatttgctt tacttgaaat ctggcaattc 1800

tggacccaca ttcccagaag ttaacaaaga aagaggggct gagtaggggc cacgcttagt 1860

cccgtttagg ctattcctta gctcctgtag gcattggaat ttgcgccctc gggcatcaaa 1920

tttccttatt ttggaagtga gaaaacagag acccagcata gcagagtgac ttcctctaag 1980

ggatccaggt atcccagccc taacctcttt ccataccgca ctgcccagga tacaaaagca 2040

tcctaaaact cctgatttgt catgactgca catatgcgca catatgtcaa agcgagtgtg 2100

ggtttggaga ctctagagaa atgaaccagc cataaatcat tttcaagcta aggaaaaaag 2160

acttaaatat atgaatggga ttagtacaaa aggatcttgt caattaaaaa attccaggac 2220

aggctgggca cggtggttcg cgcctgtaat cccagcactt tgggaggcca aggcgggcgg 2280

atcacaaggt caggagtttg agaccagcct ggccgatatg gagaaaccct gtctctacta 2340

aaaatacaaa aattagctgg gcgtggtggt gggcacctgt agtcccagct acttgggagc 2400

tgaggcagga gaatccatca tgccactgca ctccagtctg ggcaaaagag ggagactccg 2460

tctcacgaaa aacaaacaaa caaacaacaa acaaacaaaa aaaacccaag gaaaatgaca 2520

tttctgttgc gtttggtcag aag 2543

<210> 2

<211> 1109

<212> RNA

<213> homo sapiens

<400> 2

aauuuccuua uuuuggaagu gagaaaacag agacccagca uagcagagug acuuccucua 60

agggauccag guaucccagc ccuaaccucu uuccauaccg cacugcccag gauacaaaag 120

cauccuaaaa cuccugauuu gucaugacug cacauaugcg cacauauguc aaagcgagug 180

uggguuugga gacucuagag aaaugaacca gccauaaauc auuuucaagc uaaggaaaaa 240

agacuuaaau auaugaaugg gauuaguaca aaaggaucuu gucaauuaaa aaauuccagg 300

acaggcuggg cacggugguu cgcgccugua aucccagcac uuugggaggc caaggcgggc 360

ggaucacaag gucaggaguu ugagaccagc cuggccgaua uggagaaacc cugucucuac 420

uaaaaauaca aaaauuagcu gggcguggug gugggcaccu guagucccag cuacuuggga 480

gcugaggcag gagaauccau caugccacug cacuccaguc ugggcaaaag agggagacuc 540

cgucucacga aaaacaaaca aacaaacaac aaacaaacaa aaaaaaccca aggaaaauga 600

cauuucuguu gcguuugguc agaaggccca uugcaaucug ggguucuuau ucuaccuguu 660

cagggaaaua cgaaguccag augaaugugu auuccaauuc ugacucaggc uugguuucac 720

uuucuaaauu gcucagcagg aaucugauaa gaugcugaga aaagaguaaa aagaaaaaau 780

uggagggugg ggcggggaga gccagaaaua gcugguuuag gggaggccuc cugggauuac 840

caugaaccaa ugcaguaauu aauaauauga ggaacagaau cuauccgaau guuaauuaaa 900

guguuuccuc cucccuuacu cacucucucu agggcccccu ggaggugcuc auugaauuua 960

cuuuucccag gccauucucc gcuccccuag acagaucauc ugaggugggg ugcggggguu 1020

gggcgggaaa acagucucug uuaccagaga aucagaaaaa caaaagcugg ccgggcucgg 1080

uggcucacgc cuguaauccc agcacuuug 1109

<210> 3
<211> 48
<212> DNA
<213> artificial

<220>
<223> shRNA against human eRNA-22

<400> 3
ggaaatacga agtccagatg actcgagtca tctggacttc gtatttcc 48

<210> 4
<211> 48
<212> DNA
<213> artificial

<220>
<223> shRNA against human eRNA 22

<400> 4
gctcagcagg aatctgataa gctcgagctt atcagattcc tgctgagc 48

<210>  5
<211>  48
<212>  DNA
<213>  artificial

<220>
<223>  sh RNA against human eRNA 22

<400>  5
ggaacagaat ctatccgaat gctcgagcat tcggatagat tctgttcc                48

<210>  6
<211>  1103
<212>  DNA
<213>  Mus musculus

<400>  6
ggtatgcagc cccggctgga ctcaaaatca caacctgctt gcctccgcat cctgaggggt      60

agggttacag gtgtgcacct tcacaccctg tggcatccct tattttagag aaaactgaga     120

cccgaggcag taaacggact ccctctgagt gaccaaactc tagccacttt ctaccccatt     180

gtcccctccc ctacggcttc acactagcac acagggacag gagtgaaggg tgaaggcagg     240

cttgaagacc ccacggcaca tgcgccactt aatcacctta aaactgggcg tggtggcaca     300

ttcctggaag ccctggagac aggaagaagt ttaagatcat ccttggctaa aaataagttt     360

gaggccagcc ttcatgacat gcgaccctgt ctcaaagcaa aaataaaaca caaccaacca     420

accaaccacc accaccacca cccaaaaaaa ccaaaaaaca aaacccacat gtttttaaaa 480

acaagatttc tcctcccttc atcactttca agcaaagggc aaaaaaagta aactaaggta 540

tggactagat taccacaaaa ggttttttgt gagttagaaa cttccagaac aataaggttt 600

ctgcccctct cgctcacaag ttttgcgcat tccactctgt ggcactttct acgtgtttaa 660

ggaaaccctt agtccagatg caactttgtt ccaattctga gtcagccttg gtttcacttt 720

caaatcactg cgtagcagtc tggtaaaacg ttgagtaagg agtaagaagg gaaagctgga 780

gggtggggct gggcggggcg agtgagcaat agcttgttca gaggatgcca ccggggagta 840

acaggcagta attaataaca tggcggtcag agtcacagcc ctagtgttaa ttaaagagct 900

tcctccttgc tggcttattc tctagggccc ccttggagga gctcacggac tttaatgctt 960

ctgcctgagg ttgttcgcat ccgttggtgg ggactggaag gccaggtcct cagagaatca 1020

gaaaacaaca ccacaaaccc aataaccgcc cctgggcagc tggactggct tcagggaggt 1080

acattgacct gtacttccaa aag 1103

<210> 7

<211> 48

<212> DNA

<213> artificial

<220>

<223> shRNA encoding sequence

<400> 7

ggcacatgcg ccacttaatc actcgagtga ttaagtggcg catgtgcc 48

<210>  8
<211>  48
<212>  DNA
<213>  artificial

<220>
<223>  shRNA encoding sequence

<400>  8
gcttcctcct tgctggctta tctcgagata agccagcaag gaggaagc                    48

<210>  9
<211>  48
<212>  DNA
<213>  artificial

<220>
<223>  sh-RNA encoding DNA sequence

<400>  9
ggtacattga cctgtacttc cctcgaggga agtacaggtc aatgtacc                    48